# EUROPÄISCHE PATENTSCHRIFT

(11) **EP 1 244 420 B1**
(45) Veröffentlichungstag und Bekanntmachung des Hinweises auf die Patenterteilung: **16.02.2005**
(21) Anmeldenummer: 00981380.9
(22) Anmeldetag: 15.12.2000
(51) Int. Cl.: A61K 7/06

(54) **HAUT- UND HAARPFLEGEMITTEL**
HAIR AND SKIN CARE AGENTS
PRODUITS POUR LES SOINS DE LA PEAU ET DES CHEVEUX

(30) Priorität: 24.12.1999 DE 19962877
(43) Veröffentlichungstag der Anmeldung: 02.10.2002
(73) Patentinhaber: Henkel Kommanditgesellschaft auf Aktien, 40589 Düsseldorf (DE)
(72) Erfinder: BOSSMANN, Britta, 40699 Erkrath (DE); BERNECKER, Ullrich, 52393 Hürtgenwald (DE); HOLLENBERG, Detlef, 40699 Erkrath (DE)
(86) Internationale Anmeldenummer: PCT/EP2000/012817
(87) Internationale Veröffentlichungsnummer: WO 2001/047480

(56) Entgegenhaltungen:
- WO-A-99/11226
- DE-A- 19 710 154
- US-A- 5 387 374
- US-A- 5 945 093

## Beschreibung

Die Erfindung betrifft kosmetische Mittel zur Pflege der Haut und des Haars, die als Wirkstoffe zur Verbesserung der kosmetischen Eigenschaften der Keratinoberfläche eine Kombination aus einem Dialkylcarbonat und einem Aminoalkylamid enthalten.

Zahlreiche kosmetische Zubereitungen haben die Aufgabe, die Oberfläche der Haut weich und glatt und das Haar glänzend und gut kämmbar zu machen. In der Regel wird dieses Ziel durch die Einarbeitung verschiedener kosmetischer Ölkomponenten erreicht. Als besonders effektiv haben sich vor allem die Silikonöle erwiesen, die deshalb eine Sonderstellung in der Gruppe der kosmetischen Ölkomponenten einnehmen.

Es ist aber bekannt, daß die Silikonöle wegen ihrer starken Grenzflächenaktivität und Spreitwirkung sowie ihrer starken Hydrophobie nicht nur sehr leicht auf Haut und Haare aufziehen, sondern sich z. B. durch Waschen nur noch unvollständig von der Keratinoberfläche entfernen lassen, so daß es nach mehrmaliger Anwendung zur Akkumulation, z. B. am Haar zum sogenannten "build up"-Effekt kommt. Dieser führt zu einer Beeinträchtigung des kosmetischen Aussehens und der Wirkung weiterer Behandlungen, z. B. der Färbe- oder Dauerwellbehandlung.

Es hat daher nicht an Versuchen gefehlt, Wirkstoffe zur Verbesserung der kosmetischen Eigenschaften der Keratinoberfläche zu finden, die diese nachteilige Wirkung nicht aufweisen.

Dialkylcarbonate sind z. B. aus WO 92/22282 A1 als kosmetische Ölkomponenten bekannt. In DE 197 10 154 C2 und WO 97/47281 A1 wird ihre Eignung zur Verbesserung von Griff und Glanz der damit behandelten Haare beschrieben.

Aminoalkylamide sind z. B aus US 5,198,209 als kationische oberflächenaktive Stoffe in Kombination mit Silikonen, anionischen Tensiden und N-Alkyl-2-pyrrolidinonen als Komponenten in konditionierenden Shampoos bekannt.

Es wurde nun überraschend festgestellt, daß die konditionierenden Eigenschaften beider Stoffe sich in der Kombination nicht nur ergänzen, sondern daß die kosmetischen Eigenschaften solcher Zusammensetzungen, die beide Komponenten in wirksamen Mengen enthalten, durch keine der beiden Substanzen allein erreicht werden können. Dies gilt insbesondere für die Kämmbarkeit, den Griff und die statische Aufladung des mit solchen Zusammensetzungen behandelten Haars.

Gegenstand der Erfindung sind daher kosmetische Zusammensetzungen zur Pflege der Haut oder des Haars, die eine Wirkstoffkombination zur Verbesserung der kosmetischen Eigenschaften der Keratinoberfläche enthalten und die als Wirkstoffe wenigstens (A) ein Dialkylcarbonat der Formel R¹OC(O)OR², in der R¹ und R² unabhängig voneinander lineare oder verzweigte Alkylgruppen mit 6 - 22 C-Atomen sind, und wenigstens (B) ein Aminoalkylamid der Formel R³CONH(CH₂)ₙ-NR⁴R⁵, in der R³CO eine lineare Acylgruppe mit 6 - 22 C-Atomen, R⁴ und R⁵ unabhängig voneinander Alkyl-, Hydroxyalkyl- oder Aminoalkylgruppen mit bis zu 6 C-Atomen oder gemeinsam mit dem N-Atom einen 5- oder 6-gliedrigen Ring bilden, und n Zahlen von 2 bis 6 sind, enthalten.

Als kosmetische Zusammensetzungen im Sinne der vorliegenden Erfindung gelten alle Zusammensetzungen und Darreichungsformen, die zur Aufbringung der Substanzen auf die Haut oder auf das Haar geeignet sind. Solche geeigneten Zusammensetzungen können z. B. wasserfreie Öle, Gele, Aerosole oder Stiftzubereitungen sein. Es können aber auch wasserhaltige Zubereitungen, z. B. Emulsionen oder Schüttelmixturen (instabile Emulsionen), Emulsionsschäume in Pumpspray oder Aerosolverpackung, Emulsionsstifte oder Emulsionsgele sein.

In wasserfreien Zubereitungen ist das Dialkylcarbonat (A) bevorzugt in einer Menge von 1 - 90 Gew.-% enthalten. Das Aminoalkylamid (B) ist in wasserfreien Zubereitungen bevorzugt in einer Menge von 0,1 - 10 Gew.-% enthalten. Daneben können wasserfreie Zubereitungen weitere Öl-, Fett- oder Wachskomponenten, Lösungsmittel, Treibgase und Duftstoffe enthalten.

Bevorzugt liegen die erfindungsgemäßen Zubereitungen in Form einer Öl-in-Wasser-Emulsion vor, deren Ölphase das Dialkylcarbonat (A) enthält.

In solchen Emulsionen ist das Dialkylcarbonat (A) bevorzugt in einer Menge von 1 - 90 Gew.-% der Ölphase, insbesondere von 5 - 50 Gew.-% der Ölphase, enthalten.

Das Aminoalkylamid (B) ist in solchen Emulsionen bevorzugt in einer Menge von 0,1 - 2 Gewichtsteilen pro Gewichtsteil des Dialkylcarbonats enthalten. Da die Aminoalkylamide nur wenig wasserlöslich sind, aber eine zur Salzbildung befähigte basische Aminogruppe aufweisen, liegen sie im alkalischen Medium als Bestandteil der Ölkomponente vor.

Es ist aber bevorzugt, daß die erfindungsgemäßen Zusammensetzungen eine Säure in einer Menge enthalten, die das Aminoalkylamid wenigstens teilweise in ein wasserlösliches Salz überführt. Bevorzugt soll durch den Säurezusatz die wäßrige Phase der Zusammensetzung einen pH-Wert von 2 - 5 annehmen.

Bevorzugt geeignete Säuren sind z. B. die Hydroxycarbonsäuren mit 2 - 6 C-Atomen, wie z. B. Glycolsäure, Milchsäure, Äpfelsäure, Citronensäure und Weinsäure. Andere grundsätzlich geeignete Säuren sind Essigsäure, Gluconsäure, Ascorbinsäure und anorganische Säuren, wie z. B. Phosphorsäure, Schwefelsäure oder Salzsäure.

Von den Dialkylcarbonaten der Formel R¹-O-C(O)-O-R² sind insbesondere die symmetrischen Dialkylcarbonate geeignet, in deren Formel R¹ = R² ist und die durch Umesterung von niedermolekularen Dialkylcarbonaten, z. B. Diethylcarbonat, mit Alkoholen mit 6 - 22 C-Atomen erhältlich sind. Ein besonders gut geeignetes Produkt ist z. B. das Di-n-octylcarbonat.

Weitere anwendungstechnisch interessante Dialkylcarbonate weisen verzweigte Alkylgruppen auf Ein großtechnisch verfügbares Produkt dieser Art ist z. B. das Di-(2-hexyldecyl)-carbonat, das durch Umesterung von Diethylcarbonat mit einem Guerbet-Alkohol, 2-Hexyl-decanol, gewonnen wird und gelegentlich als "Guerbetcarbonat" bezeichnet wird.

Die Aminoalkylamide sind technisch durch Amidbildung aus Fettsäuren mit 6 - 22 C-Atomen oder deren Estern und N,N-disubstituierten Alkylendiaminen der Formel H₂N-(CH₂)ₙ-NR⁴R⁵ zugänglich. Die Verbindungen dienen üblicherweise als Ausgangsprodukte zur Erzeugung von quartären Ammoniumverbindungen oder Betaintensiden. Im Handel erhältlich ist z. B. das sog. Dimethylaminopropyl-Stearamid unter der Handelsbezeichnung Tego® Amid S18 (Tego Cosmetics) oder das Isostearamidopropylmorpholin unter der Handelsbezeichnung Incromate® ISML (Croda Inc.). Weitere geeignete Aminoalkylamide sind z. B. Stearinsäuredihydroxyethylaminoethylamid oder Myristinsäure-dimethylaminopropylamid.

Die erfindungsgemäßen Zusammensetzungen können zwar prinzipiell auch mit stark schäumenden Tensiden zu emulsionsförmigen Shampoos und Körperreinigungsmitteln verarbeitet werden. Bevorzugt aber werden die erfindungsgemäßen Zubereitungen zur Pflege, Festigung, Färbung oder Aufhellung des Haars verwendet. Solche Zubereitungen sind z. B. Haaröle, Haargele, Haarwässer, haarkonditionierende Cremes und Emulsionen, Haarkuren. emulsionsförmige Haarfestiger und Schaumfestiger, emulsionsförmige Färbe- und Aufhellungsmittel sowie Dauerwellfixierlotionen.

Neben den kennzeichnenden Wirkstoffkombinationen können in solchen Zubereitungen die für die genannten Mittel üblichen Bestandteile enthalten sein.

Solche fakultativen Komponenten sind z. B. oberflächenaktive Verbindungen, insbesondere nichtionogene, ampholytische, zwitterionische und kationische oberflächenaktive Stoffe. Geeignete nichtionische Tenside sind z. B. die Anlagerungsprodukte von Ethylenoxid an Fettstoffe mit beweglichen Wasserstoffatomen, z. B. an Fettsäuren, an Fettalkohole, an Fettsäurealkanolamide, an Fettsäuremonoglyceride, an Sorbitanfettsäureester, an Methylglucosid-fettsäureester, an Rizinusöl. an Polypropylenglycole, an Propylenglycolmonofettsäureester und andere Fettstoffe. Weitere nichtionogene Tenside sind die Alkyl- und/oder Alkenyloligoglucoside, die Fettsäure-N-alkylglucamide, Tri-(polyalkoxy)-alkylphosphate und Aminoxid-Tenside. Geeignete ampholytische Tenside sind z. B. N-Alkylaminocarbonsäuren; geeignete zwitterionische Tenside sind z. B. die Betaintenside wie z. B. Kokosalkyl-dimethylammonium-glycinat.

In einer besonders bevorzugten Ausführungsform enthalten die erfindungsgemäßen Zubereitungen zur Haarbehandlung zusätzlich eine oberflächenaktive quartäre Ammonium-, Pyridinium- oder Imidazoliniumverbindung oder ein wasserlösliches kationisches Polymer in einer Menge von 0,1 - 5 Gew.-% der Zusammensetzung.

Die kationischen Tenside vom Typ der quartären Ammonium-, Pyridinium- oder Imidazoliniumverbindungen zeichnen sich dadurch aus, daß eine oder zwei lipophile Alkyl- oder Acylgruppen mit 10 - 22 C-Atomen mit dem Stickstoffatom einer quartären Ammonium-, Pyridinium- oder Imidazoliniumstruktur verbunden sind. Typische Beispiele solcher quartärer Ammonium-Tenside sind z. B. das Cetyl-dimethyl-benzylammoniumchlorid, das Lauryl-trimethyl-ammoniumchlorid, das Di-stearoyloxyethylhydroxyethyl-methyl-ammoniumchlorid, das Cetyl-pyridinium-chlorid oder das Methyl-2-stearyl-3-stearoylamidoethyl-imidazolinium-methoxysulfat.

Geeignete wasserlösliche kationische Polymerisate sind z. B. die Copolymeren von Diallylammoniumsalzen und Acrylamiden, quatemierte Vinylpyrrolidin-Vinylimidazol-Polymere, Kondensationsprodukte von Polyglycolen und Aminen, Polyethylenimin, kationische Silikonpolymere, Copolymere von Adipinsäure und Dimethylaminohydroxypropyl-diethylentriamin, Copolymere von Acrylsäure und Dimethyldiallylammoniumchlorid. Solche wasserlöslichen kationischen Polymerisate sind z. B. unter den Warenzeichen Merquat® 550, Gafquat®, Luviquat® oder Cartaretine® im Handel erhältlich.

Geeignet sind aber auch wasserlösliche Derivate von polymeren Naturstoffen wie Cellulose, Stärke, Guar, Chitin oder Proteinen, die sich durch wiederkehrende kationische Gruppen in oder an der Polysaccharid- oder Peptidkette auszeichnen. Solche Produkte sind z. B. unter den Warenzeichen Polymer® JR400, Lamequat® L oder Jaguar® im Handel.

Neben dem Dialkylcarbonat können die erfindungsgemäßen Zubereitungen auch andere Ölkomponenten enthalten. Obwohl Silikonöle die oben beschriebenen Nachteile aufweisen, ist ihre zusätzliche Anwesenheit in den Zubereitungen gemäß der Erfindung nicht ausgeschlossen. Dies gilt besonders für niedrigsiedende cyclische Silikone wie z. B. das Cyclopentasiloxan. Bevorzugt sind aber andere Öl- und Fettkomponenten enthalten, insbesondere solche, die das Haar auch bei häufiger Anwendung nicht belasten.

Geeignete Ölkomponenten sind z. B. pflanzliche und tierische oder synthetische Triglyceridöle, natürliche oder synthetische Wachsester, z. B. Jojobaöl oder Cetylpalmitat, Paraffinöle und synthetische Kohlenwasserstoffe wie z. B. 1,3-Dioctylcyclohexan, Squalan, Fettsäureester wie Isopropylmyristat, Butylstearat oder n-Hexyllaurat und andere flüssige Ester.

Besonders geeignete Ölkomponenten, die zusammen mit der erfindungsgemäßen Wirkstoffkombination hervorragende Pflegeeigenschaften in haarkosmetischen Produkten aufweisen, sind die Dialkylether mit 12 - 24 C-Atomen, insbesondere die symmetrischen Di-n-alkylether. Eine besonders bevorzugte Ölkomponente dieser Art ist der Di-n-octylether, der unter dem Warenzeichen Cetiol® OE im Handel ist.

Schließlich können die erfindungsgemäßen Zubereitungen auch weitere Hilfs- und Zusatzstoffe enthalten. Solche Stoffe sind z. B. antimikrobielle, deodorierende und konservierende Stoffe, Antischuppenmittel, Komplexbildner, Puffersubstanzen, Farbstoffe. Trübungsmittel, Duftstoffe, Lösungsmittel sowie haut- und haarkosmetische Wirkstoffe. wie z. B. Panthenol, Vitamine, Sebostatika, UV-Filtersubstanzen.

Die folgenden Beispiele sollen den Gegenstand der Erfindung näher erläutern, ohne ihn hierauf zu beschränken:

### Beispiele

Es werden Haarpflegeemulsionen (Haarspülungen) gemäß folgender Zusammensetzung hergestellt:

| | **1** | **2** | **V1** | **V2** | **V3** | **V4** | **V5** | **V6** |
|---|---|---|---|---|---|---|---|---|
| (1) Lanette® O | 2,7 | 2,7 | 2,7 | 2,7 | 2,7 | 2,7 | 2,7 | 2,7 |
| (2) Isopropylmyristat | 1,0 | 1,0 | 1,0 | 1,0 | 1,0 | 1,0 | 1,0 | 1,0 |
| (3) Cetiol® OE | 0,75 | - | 0.75 | 0,75 | - | - | 0,75 | - |
| (4) Dioctylcarbonat | 0,75 | 0,75 | - | 0,75 | - | 0,75 | - | - |
| (5) Tego® Amid S18 | 0,8 | 0,8 | 0,8 | - | 0,8 | - | - | - |
| (6) Rewoquat® W75 PG | 2,5 | 2,5 | 2,5 | 2,5 | 2,5 | 2,5 | 2,5 | 2,5 |
| (7) Dehyquart® F75 | 1,0 | 1,0 | 1,0 | 1,0 | 1,0 | 1,0 | 1,0 | 1,0 |
| (8) Phenoxyethanol | 0,4 | 0,4 | 0,4 | 0,4 | 0,4 | 0,4 | 0,4 | 0,4 |
| (9) PHB-Methylester | 0,2 | 0,2 | 0,2 | 0,2 | 0,2 | 0,2 | 0,2 | 0,2 |
| (10) Wasser | ad 100 | ad 100 | ad 100 | ad 100 | ad 100 | ad 100 | ad 100 | ad 100 |
| (11) Citronensäure | 0,2 | 0,2 | 0,2 | 0,2 | 0,2 | 0,2 | 0,2 | 0,2 |
| (12) Panthenol | 0,15 | 0,15 | 0,15 | 0,15 | 0,15 | 0,15 | 0,15 | 0,15 |
| (13) Parfüm | 0,3 | 0,3 | 0,3 | 0,3 | 0,3 | 0,3 | 0,3 | 0,3 |
| pH-Wert | 2,6 - 4,5 | | | | | | | |
| Naßkämmbarkeit | 1- | 1+ | 1- | 2- | 2- | 2 | 2+ | 3- |
| Griff (naß) | 1- | 1- | 1- | 2 | 2 | 1- | 1- | 2 |
| Trockenkämmbarkeit | 1- | 1- | 2+ | 3 | 2- | 1- | 1- | 3 |
| Griff (trocken) | 1 | 1 | 1 | 2- | 3 | 1 | 1 | 2 |
| Statische Aufladung | 1 | 1 | 2 | 1 | 1 | 2 | 2 | 1 |
| Belastung | keine | keine | keine | keine | keine | keine | keine | keine |

### Verwendete Handelsprodukte:

| | |
|---|---|
| Lanette®O | Cetyl-Stearylalkohol (1 : 1) |
| Cetiol®OE | Di-n-octylether |
| Tego Amid® S18 | Stearamidopropyl-dimethylamin (Stearinsäure-N-(3-dimethylamino)-propylamid) |
| Rewoquat® W75 PG | Quatemium-87, Propyleneglycol (Methyl-2-norpalmalkyl-3-palmfettsäureamidoethylimidazolinium-methosulfat) (mit 25 % Propylenglycol) |
| Dehyquart® F75 | Distearoylethyl-hydroxyethyl-methyl-ammonium-Methosulfat (mit 25 % Cetyl-/Stearylalkohol) |

### Herstellungsweise:

Die Bestandteile der Ölphase (1) bis (9) wurden im Ansatzbehälter auf 80° C erwärmt und gemischt.

Die Citronensäure wurde in Wasser gelöst und die Lösung auf 80° C erwärmt. Die Ölphase wurde unter Rühren in die erwärmte Wasserphase einemulgiert. Nach Abkühlung des Ansatzes auf 35° C wurden Panthenol und Parfümöl zugesetzt und unter Rühren eingemischt.

### Anwendungstechnische Prüfungen:

### 1. Bestimmung der Naßkämmbarkeit

### 1.1 Vorbehandlung der Prüfsträhnen

Trockene Haarsträhnen von ca. 2 g Gewicht (Fischbach und Miller, Typ 6923) wurden einmal mit 32 g Blondiermittel (Handelsprodukt: Poly Blond Medium Aufheller) eine halbe Stunde lang blondiert. Nach dem Auswaschen der Blondiermischung wurden die Haarsträhnen unmittelbar ohne Zwischentrocknung einer Dauerwellbehandlung mit dem Marktprodukt Poly Lock Normal unterzogen. Die Einwirkdauer von Wellkomponente und Fixierkomponente betrugen dabei 30 bzw. 15 Minuten. Nach dem Ausspülen der Fixierkomponente wurden die Strähnen getrocknet und mindestens zwei Tage bei Umgebungsbedingungen konditioniert.

### 1.2 Bestimmung der Kämmbarkeit

Die gespülte Haarsträhne wurde vor der Bestimmung mit 0,2 ml einer 50 %igen wäßrigen Lösung von Texapon® N25 (28 %ige Lösung von Natriumlaurylethersulfat in Wasser) intensiv shampooniert und anschließend ausgespült. Danach wurde 1 g der zu prüfenden Zubereitung gleichmäßig in das Haar massiert. Auszuspülende Produkte wurden eine Minute auf dem Haar belassen und dann sorgfältig ausgespült. Nach dem Einmassieren (bei auf dem Haar verbleibenden Produkten) bzw. nach dem Ausspülen (bei abzuspülenden Produkten) wurde das Haar mit einem feinzinkigen Hartgummikamm gekämmt und der Kämmwiderstand subjektiv beurteilt. Vergleichszubereitungen wurden anschließend an der gleichen Strähne in gleicher Weise geprüft. Die Beurteilung wurde entsprechend einem Beurteilungssystem von 1 (= sehr gut) bis 5 (= sehr schlecht) abgegeben.

Die Ergebnisse sind der Tabelle zu entnehmen.

### 2. Bestimmung der Trocken-Kämmbarkeit

Die Bestimmung wurde analog wie unter 1.2 durchgeführt, jedoch wurde das Haar vor der Kämmbarkeitsbestimmung 2 Stunden im warmen Luftstrom (30° C, 20 % rel. Feuchtigkeit) getrocknet und 12 Stunden bei 30° C, 20 % rel. Feuchte konditioniert.

### 3. Bestimmung der elektrostatischen Aufladung

Die elektrostatische Aufladung wurde gleichzeitig mit der Trockenkämmbarkeit unter Zulassung elektrostatischer Aufladung untersucht. Die Haarsträhnen wurden hierzu innerhalb von 10 sec. 20 mal mit einem feinzinkigen Hartgummikamm durchgekämmt und das durch die statische Aufladung verursachte Aufspreizen der Haarsträhne beurteilt.

Die Bewertung erfolgte durch Benotung nach einem dreiteiligen Schema von 1 (sehr wenig) bis 3 (starke Aufladung).

### 4. Griffbenotung

Die Beurteilung des Griffs der nassen und der trockenen Haare erfolgte durch manuelle sensorische Prüfung und Beurteilung nach einem fünfteiligen Schema von 1 (sehr weich) bis 5 (strähnig).

### 5. Belastbarkeit

Die Belastung wurde an nicht vorbehandelten aber im übrigen wir unter 1.2 shampoonierten und wie unter 2. getrockneten Haarsträhnen durch manuelle sensorische Prüfung auf Rückstände beurteilt.

## Patentansprüche

1. Kosmetische Zusammensetzung zur Pflege der Haut oder des Haars, die eine Wirkstoffkombination zur Verbesserung der kosmetischen Eigenschaften der Keratinoberfläche enthält, **dadurch gekennzeichnet, daß** als Wirkstoffe wenigstens
(A) ein Dialkylcarbonat der Formel R¹OC(O)OR², in der R¹ und R² unabhängig voneinander lineare oder verzweigte Alkylgruppen mit 6 - 22 C-Atomen sind und wenigstens
(B) ein Aminoalkylamid der Formel R³CONH(CH₂)ₙ - NR⁴R⁵, in der R³CO eine lineare Acylgruppe mit 6 - 22 C-Atomen, R⁴ und R⁵ unabhängig voneinander Alkyl-, Hydroxyalkyl- oder Aminoalkylgruppen mit bis zu 6 C-Atomen sind oder gemeinsam mit dem N-Atom einen 5- oder 6-gliedrigen Ring bilden, und n eine Zahl von 2 bis 6 ist, enthalten sind.

2. Zusammensetzung nach Anspruch 1, **dadurch gekennzeichnet, daß** sie in Form einer Öl-in-Wasser-Emulsion vorliegt, deren Ölphase das Dialkylcarbonat (A) enthält.

3. Zusammensetzung gemäß Patentanspruch 2, **dadurch gekennzeichnet, daß** das Dialkylcarbonat (A) in einer Menge von 1 - 90 Gew.-% der Ölphase, insbesondere von 5 - 50 Gew.-% der Ölphase. enthalten ist.

4. Zusammensetzung gemäß einem der Ansprüche 1 - 3, **dadurch gekennzeichnet, daß** das Aminoalkylamid (B) in einer Menge von 0,1 - 2 Gewichtsteilen pro Gewichtsteil des Dialkylcarbonats enthalten ist.

5. Zusammensetzung gemäß einem der Ansprüche 2 - 4, **dadurch gekennzeichnet, daß** eine Säure. bevorzugt eine Hydroxycarbonsäure mit 2 - 6 C-Atomen in einer Menge enthalten ist. die der wäßrigen Phase der Zusammensetzung einen pH-Wert von 2 - 5 verleiht.

6. Zusammensetzung gemäß einem der Ansprüche 1 - 5, **dadurch gekennzeichnet, daß** zusätzlich eine oberflächenaktive quartäre Ammonium-, Pyridinium- oder Imidazoliniumverbindung oder ein wasserlösliches kationisches Polymer in einer Menge von 0,1 -5 Gew.-% der Zusammensetzung enthalten ist.

7. Verwendung der Zusammensetzung gemäß Anspruch 1 - 6 zur Pflege, Festigung, Färbung oder Aufhellung der Haare.

8. Verwendung einer Wirkstoffkombination aus (A) einem Dialkylcarbonat der Formel R¹OC(O)OR², in der R¹ und R² unabhängig voneinander lineare oder verzweigte Alkylgruppen mit 6 - 22 C-Atomen sind, und (B) einem Aminoalkylamid der Formel R³CONH(CH₂)ₙ - NR⁴R⁵, in der R³CO eine lineare Acylgruppe mit 6 - 22 C-Atomen ist, R⁴ und R⁵ unabhängig voneinander Alkyl-, Hydroxyalkyl- oder Aminoalkylgruppen mit bis zu 6 C-Atomen sind oder gemeinsam mit dem N-Atom einen 5- oder 6-gliedrigen Ring bilden, und n eine Zahl von 2 - 6 ist, zur Verbesserung der Kämmbarkeit, des Griffs und des Glanzes der damit behandelten Haare.

## Claims

1. A cosmetic skin or hair care composition containing a combination of active principles for improving the cosmetic properties of the keratin surface, **characterized in that** it contains as active principles at least (A) a dialkyl carbonate with the formula R¹OC(O)OR², in which R¹ and R² independently of one another represent linear or branched C₆₋₂₂ alkyl groups, and at least (B) an aminoalkyl amide with the formula R³CONH(CH₂)ₙ-NR⁴R⁵, in which R³CO is a linear C₆₋₂₂ acyl group, R⁴ and R⁵ independently of one another represent alkyl, hydroxyalkyl or aminoalkyl groups containing up to 6 carbon atoms or, together with the N atom, form a 5- or 6-membered ring and n is a number of 2 to 6.

2. A composition as claimed in claim 1, **characterized in that** it is present in the form of an oil-in-water emulsion of which the oil phase contains the dialkyl carbonate (A).

3. A composition as claimed in claim 2, **characterized in that** the dialkyl carbonate (A) is present in a quantity of 1 to 90% by weight, based on the oil phase, and more particularly in a quantity of 5 to 50% by weight, based on the oil phase.

4. A composition as claimed in any of claims 1 to 3, **characterized in that** the aminoalkyl amide (B) is present in a quantity of 0.1 to 2 parts by weight per part by weight of the dialkyl carbonate.

5. A composition as claimed in any of claims 2 to 4, **characterized in that** an acid, preferably a C₂₋₆ hydroxycarboxylic acid, is present in a quantity which gives the aqueous phase of the composition a pH value of 2 to 5.

6. A composition as claimed in any of claims 1 to 5, **characterized in that** a surface-active quaternary ammonium, pyridinium or imidazolinium compound or a water-soluble cationic polymer is additionally present in a quantity of 0.1 to 5% by weight, based on the composition.

7. The use of the composition claimed in claims 1 to 6 for the care, setting, coloring or lightening of the hair.

8. The use of an active-principle combination of (A) a dialkyl carbonate with the formula R¹OC(O)OR², in which R¹ and R² independently of one another represent linear or branched C₆₋₂₂ alkyl groups, and (B) an aminoalkyl amide with the formula R³CONH(CH₂)ₙ-NR⁴R⁵, in which R³CO is a linear C₆₋₂₂ acyl group, R⁴ and R⁵ independently of one another represent alkyl, hydroxyalkyl or aminoalkyl groups containing up to 6 carbon atoms or, together with the N atom, form a 5- or 6-membered ring and n is a number of 2 to 6,
for improving the combability, feel and luster of the hair thus treated.

## Revendications

1. Composition cosmétique pour les soins de la peau ou des cheveux, qui contient une combinaison de substances actives pour améliorer les propriétés cosmétiques de la surface de kératine, **caractérisée en ce que**, en tant que substances actives, y sont contenus au moins
(A) un carbonate de dialkyle, de formule R¹OC(O)OR², dans laquelle R¹ et R² sont indépendamment l'un de l'autre, des groupes alkyle linéaires ou ramifiés ayant 6 à 22 atomes de C, et au moins
(B) un amide d'aminoalkyle de formule R³CONH(CH₂)ₙ-NR⁴R⁵, dans laquelle R³CO est un groupe acyle linéaire ayant 6 à 22 atomes de C, R⁴ et R⁵ sont indépendamment l'un de l'autre, des groupes alkyle, hydroxyalkyle ou aminoalkyle, ayant jusqu'à 6 atomes de C, ou forment conjointement avec l'atome de N, un cycle à 5 ou 6 maillons, et n est un nombre de 2 à 6.

2. Composition selon la revendication 1, **caractérisée en ce qu'**elle se présente sous forme d'une émulsion huile-dans-eau, dont la phase d'huile contient le carbonate de dialkyle (A).

3. Composition selon la revendication 2, **caractérisée en ce que** le carbonate de dialkyle (A) y est contenu en une quantité de 1 à 90% en poids de la phase d'huile, en particulier de 5 à 50% en poids de la phase d'huile.

4. Composition selon l'une quelconque des revendications 1 à 3, **caractérisée en ce que** l'amide d'aminoalkyle (B) y est contenu en une quantité de 0,1 à 2 parties en poids, par partie en poids du carbonate de dialkyle.

5. Composition selon l'une quelconque des revendications 2 à 4, **caractérisée en ce qu'**un acide, de préférence un acide hydroxycarboxylique ayant 2 à 6 atomes de C, y est contenu en une quantité qui confère une valeur de pH de 2 à 5 à la phase aqueuse de la composition.

6. Composition selon l'une quelconque des revendications 1 à 5, **caractérisée en ce qu'**un composé ammonium, pyridinium ou imidazolinium, quaternaire tensio-actif ou bien un polymère cationique hydrosoluble y est contenu en sus en une quantité de 0,1 à 5% en poids de la composition.

7. Utilisation de la composition selon les revendications 1 à 6, pour les soins, la fixation, la coloration ou l'éclaircissement des cheveux.

8. Utilisation d'une combinaison de substances actives constituées de (A) un carbonate de dialkyle de formule R¹OC(O)OR², dans laquelle R¹ et R² sont indépendamment l'un de l'autre, des groupes alkyle linéaires ou ramifiés ayant de 6 à 22 atomes de C, et de (B) un amide d'aminoalkyle de formule R³CONH(CH₂)ₙ-NR⁴R⁵, dans laquelle R³CO est un groupe acyle linéaire ayant de 6 à 22 atomes de C, R⁴ et R⁵ sont indépendamment l'un de l'autre, des groupes alkyle, hydroxyalkyle ou aminoalkyle, ayant jusqu'à 6 atomes de C, ou bien forment conjointement avec l'atome de N, un cycle à 5 ou 6 maillons, et n est un nombre de 2 à 6, pour améliorer l'aptitude au peignage, le toucher et le brillant des cheveux ainsi traités.
